# EUROPÄISCHE PATENTSCHRIFT

(11) **EP 0 944 369 B1**
(45) Veröffentlichungstag und Bekanntmachung des Hinweises auf die Patenterteilung: **02.10.2002**
(21) Anmeldenummer: 96915006.9
(22) Anmeldetag: 01.05.1996
(51) Int. Cl.: A61F 2/48, A61F 2/00

(54) **ENDOSPHINKTER UND SET ZUM FREIGEBBAREN VERSCHLIESSEN DER URETHRA**
ENDOSPHINCTER AND SET FOR RELEASABLY CLOSING THE URETHRA
ENDOSPHINCTER ET JEU D'OCCLUSION OUVRABLE DE L'URETRE

(30) Priorität: 05.05.1995 DE 29507519 U
(43) Veröffentlichungstag der Anmeldung: 29.09.1999
(73) Patentinhaber: Prof. Dr. Chaussy, Christian, 82064 Strasslach (DE); Dr. Med. Thürhof, Stefan, 81547 München (DE)
(72) Erfinder: Prof. Dr. Chaussy, Christian, 82064 Strasslach (DE); Dr. Med. Thürhof, Stefan, 81547 München (DE)
(74) Vertreter: Dipl.-Ing. Heiner Lichti Dipl.-Phys. Dr.rer.nat. Jost Lempert Dipl.-Ing. Hartmut Lasch
(86) Internationale Anmeldenummer: EP9601805
(87) Internationale Veröffentlichungsnummer: WO96034582

(56) Entgegenhaltungen:
- EP-A- 0 265 207
- EP-A- 0 575 719
- EP-A- 0 596 145
- EP-A- 0 622 059
- WO-A-93/10845
- WO-A-93/13824
- DE-U- 29 507 519
- FR-A- 2 595 564
- US-A- 4 850 963
- US-A- 4 932 938

## Beschreibung

Die Erfindung betrifft einen künstlichen Endosphinkter für die Urethra nach dem Oberbegriff des Anspruchs 1 und ein Set zum freigebbaren Verschließen der Urethra nach dem Oberbegriff des Anspruchs 20.

Die US-A-4,932,938 zeigt einen gattungsgemäßen künstlichen Endosphinkter. Der eigentliche Endosphinkter wird durch ein Halteteil innerhalb der Blase gehalten, das den natürlichen Ausgang von der Blase zur Urethra sperrt. Das den Urin ableitende Führungsteil ist durch das Halteteil geführt und endet oberhalb desselben. In dem Bereich oberhalb des Halteteils ist eine Einlaßöffnung ausgebildet. Die Einlaßöffnung weist daher einen erheblichen vertikalen Abstand vom natürlichen Auslaß der Blase zur Urethra auf. Dies bedingt, daß bei Entleerung der Blase durch Betätigung des Ventils immer ein erheblicher Anteil an Restflüssigkeit in der Blase zurückbleibt. Dies ist nachteilig.

Der künstliche Endosphinkter nach der WO-A-9310845 weist ein katheterähnliches Rohr auf, das sich durch die gesamte Urethra erstreckt. Das rückwärtige Ende ist flexibel ausgebildet und in der dargestellten Figur eingerollt. Aufgrund der Flexibilität kann es aber keinen sicheren Halt gegen Herausrutschen des Endosphinkters gewährleisten. Gegen ein Hochbewegen des Endosphinkters in der Urethra (also auf die Blase zu) ist der Endosphinkter nach der WO-A-9310845 durch einen das vordere Ende des Penis überragenden und abdeckenden, erweiterten Kopf gesichert, der an seinem rückwärtigen Ende (also dem Peniskopf zugewandt) Versteifungsrippen aufweist. Letztere sind nachteilig; sie können Entzündungen hervorrufen. Darüber hinaus ist diese Ausgestaltung mit dem Haltekopf oder -flügeln aus offensichtlichen Gründen, die hier nicht weiter vertieft werden müssen, zumindest unangenehm. Gleiches gilt auch hinsichtlich des Umstandes, daß der Endosphinkter bzw. der Kanal sich durch die gesamte Urethra erstreckt und nicht lediglich über eine Länge, die zur Festlegung und zur Funktion erforderlich ist. Darüber hinaus ist durch die Ausbildung und Anordnung der Einlaßöffnungen im in der Blase befindlichen Teil des Endosphinkters ebenfalls nicht sichergestellt, daß der Urin bei Öffnen des Ventils vollständig abfließt.

Der Erfindung liegt die Aufgabe zugrunde, einen künstlichen Sphinkter zu schaffen, der einfach in der Urethra plaziert und einfach bedient werden kann. Aufgabe der Erfindung ist weiterhin die Schaffung von Sets zum freigebbaren Verschließen der Urethra mittels eines solchen Sphinkters sowie Verfahren zum Einsetzen des Sphinkters.

Erfindungsgemäß wird die genannte Aufgabe zunächst durch einen gattungsgemäßen künstlichen Endosphinkter für die Urethra mit den kennzeichnenden Merkmalen des Anspruchs 1 gelöst.

Der erfindungsgemäße künstliche Endosphinkter wird durch die Urethraöffnung in dieselbe bis zu einer Positionierstelle, vorzugsweise im Bereich des zu ersetzenden natürlichen Sphinkters, d.h. im Bereich des Beckenbodens, eingebracht und mittels des Verankerungsteils dort verankert. Das im Ventilkörper befindliche selbstschließende Ventil wird durch direkte Betätigung von außen geöffnet, indem im Bereich des Ventils von außerhalb radialer Druck auf den Penis ausgeübt wird; hierzu drückt der Patient beispielsweise von oben und unten gegen den Penis. Er kann so das Ventil öffnen und damit den Urinfluß freigeben. Wenn kein äußerer Druck ausgeübt wird, bleibt das Ventil geschlossen, so daß der Urin in der Harnblase zurückgehalten wird.

Bei einer Ausgestaltung mit einem Verankerungsteil zum Festlegen des Ventilkörpers in der Urethra ist in bevorzugter Ausbildung vorgesehen, daß Verankerungsteil und Ventilkörper lösbar miteinander verbunden sind. Bei dieser Ausgestaltung können Verankerungsteil und Ventilkörper separat nacheinander in die Urethra eingeführt werden, so daß zunächst nach Einführen des Verankerungsteils sich über diesem ein Epithel bilden kann, bevor dann der Ventilkörper im Verankerungsteil festgelegt wird. Weiterhin weist diese Ausgestaltung den Vorteil auf, daß der Ventilkörper, beispielsweise wenn das Ventil inkrustiert, entfernt werden kann, das notwendigerweise epithalisierte Verankerungsteil aber in der Urethra verbleiben kann und ein neuer Ventilkörper eingesetzt und im Verankerungsteil festgelegt werden kann. Alternativ hierzu kann aber auch vorgesehen sein, daß Verankerungsteil und Ventilkörper derart fest miteinander verbunden sind, daß sie nicht ohne Beschädigung voneinander trennbar sind.

Der Haltebereich des Verankerungsteils kann schulterartig ausgebildet sein und insbesondere einen sich vom Endabschnitt trompetenartig erweiternden Erweiterungsabschnitt und einen sich vom Hauptkörper zum Erweiterungsabschnitt hin bogenförmig eingezogenen Verjüngungsabschnitt aufweisen. Dabei kann in Weiterbildung auch vorgesehen sein, daß zusätzlich ein sich teilkonisch erweiternder Zwischenabschnitt zwischen dem Erweiterungsabschnitt und dem Verjüngungsabschnitt ausgebildet ist. Hierdurch erfolgt an sich eine formschlüssige Verbindung von Verankerungsteil und Ventilkörper, die zum Trennen des letzteren von ersterem bei einer elastischen oder flexiblen Ausbildung zumindest des sich verjüngenden und verengten Bereichs des Verankerungsteils und/oder des sich erweiternden Bereichs des Ventilkörpers unter Aufwendung einer größeren Zugkraft gelöst werden kann. Die gewünschte radiale Flexibilität des Verankerungsteils kann in bevorzugter Ausgestaltung dadurch erreicht werden, daß das Verankerungsteil in seiner Mantelwand Durchbrechungen aufweist, wobei insbesondere das Verankerungsteil in seiner Einsatzkonfiguration rautenförmige Durchbrechungen aufweist. In alternativer Ausgestaltung kann aber auch vorgesehen sein, daß das Verankerungsteil schraubenfederartig ausgebildet ist.

Das Verankerungsteil besteht vorzugsweise aus einer Formgedächtnislegierung, wie einer Nickel-Titan-Legierung, die in einer bevorzugten Ausgestaltung unter der Kennzeichnung Nitinol bekannt ist. Bei einer solchen Ausgestaltung kann das Verankerungsteil in seiner sehr geringe Querabmessungen aufweisenden Tieftemperaturkonfiguration mittels geeigneter Einführelemente in die Urethra eingeführt werden und weitet sich bei der Körpertemperatur des Menschen, die wesentlich über der Übergangstemperatur des genannten Materials liegt, in seine Hochtemperaturkonfiguration mit größeren radialen Abmessungen auf, in der das Verankerungsteil an den Wandungen der Urethra anliegt und dort einen sicheren Halt gewährleistet.

Eine Weiterbildung des erfindungsgemäßen Endosphinkters sieht vor, daß der Ventilkörper einen sich vom Haltebereich erstreckenden schlauchförmigen zylindrischen Hauptkörper mit geringerem Durchmesser als der Haltebereich aufweist. Um sicherzustellen, daß am Rande des Endosphinkters kein Urin entlang der Innenwandung der Urethra hindurchläuft und um einen hinreichend sicheren Abschluß zu erreichen, sieht eine weitere bevorzugte Ausgestaltung vor, daß der zylindrische Hauptkörper nahe dem Haltebereich mit Dichtlippen versehen ist, wobei insbesondere die Dichtlippen radial sich etwas über den Haltebereich hinaus erstrecken. Gemäß einer bevorzugten Ausgestaltung ist vorgesehen, daß der Haltebereich des Ventilkörpers in radialer Richtung elastisch ausgebildet ist.

Die Erfindung beinhaltet weiterhin ein Verankerungsteil zum Festlegen eines Funktionselements wie eben eines Ventilkörpers in einem Körperkanal mit den vorstehend beschriebenen Merkmalen des Verankerungsteils des erfindungsgemäßen Endosphinkters.

Auch gehört zur Erfindung ein Set zum freigebbaren Verschließen der Urethra mit einem erfindungsgemäßen künstlichen Endosphikter und einer Einrichtung zum Einführen des Ventilkörpers in die Urethra mit den weiteren Merkmalen des Anspruchs 20. Es kann dabei weiterhin vorgesehen sein, daß der Durchmesser des vorderen Endes des Kanülenrohres kleiner ist als der Durchmesser des Endabschnittes des Verankerungsteils oder daß innerhalb des äußeren Kanülenrohrs ein den Ventilkörper umgebendes, aus dem äußeren Kanülenrohr ausfahrbares Führungsrohr angeordnet ist, das zu seinem freien Ende in flexible Finger gespalten ist, deren freie Enden aufeinander zu gebogen sind.

Ein erstes Verfahren zum Einbringen eines erfindungsgemäßen Endosphinkters mit fest miteinander verbundenem Verankerungsteil und Ventilkörper sieht vor, daß den aus einem Ventilkörper und einem fest mit diesem verbundenen Verankerungsteil bestehenden Endosphinkter aufnehmende Einführelemente einer Einrichtung zum Einführen des Endosphinkters in die Urethra durch die Urethraöffnung in die Urethra bis in den Bereich des Beckenbodens eingeführt werden und anschließend ein den Endosphinkter enthaltendes äußeres Kanülenrohr als Teil der Einführelemente in Richtung auf die Urethraöffnung relativ zu einem Gegenhalter (Anschlag) für den Endosphinkter zurückgezogen wird, wodurch zunächst ein Verankerungsteil aus dem äußeren Kanülenrohr austritt und sich aufgrund seiner Eigenelastizität bei der menschlichen Körpertemperatur radial ausdehnt und im Bereich der Pars membranacea der Urethra verankert und beim weiteren Zurückziehen des Kanülenrohrs der gesamte Endosphinkter freigegeben wird.

Ein zweistufiges Verfahren zum Einbringen eines künstlichen Endosphinkters in die Urethra sieht vor, daß ein Verankerungsteil enthaltende Einführelemente einer Vorrichtung zum Einführen des Endosphinkters in die Urethra durch die Urethraöffnung in diese bis zum Bereich des Beckenbodens eingeführt werden und ein das Verankerungsteil enthaltendes äußeres Kanülenrohr unter Gegenhaltung des Verankerungsteils durch einen Gegenhalter im Bereich des Beckenbodens freigegeben wird, so daß sich das Verankerungsteil durch elastische radiale Aufweitung bei der Körpertemperatur im Bereich der Pars membranacea der Urethra verankert und daß nach Entfernen der Einführelemente für das Verankerungsteil einen Ventilkörper enthaltende Einführelemente einer Einrichtung zum Einführen des Ventilkörpers durch die Urethraöffnung in die Urethra bis vor einen äußeren, verjüngten Endabschnitt des Verankerungsteils eingeführt wird und daß ein Halteabschnitt des Ventilkörpers durch den verjüngten Endabschnitt des Verankerungsteils hindurch in einen Haltebereich desselben eingebracht wird. In Weiterbildung ist dabei vorgesehen, daß zum Einbringen des Haltebereichs des Ventilkörpers in den Haltebereich des Verankerungsteils ein vorderer Endbereich eines den Ventilkörper aufnehmenden äußeren Kanülenrohrs der Einführelemente in den verjüngten Bereich des Verankerungsteils eingeführt wird und anschließend der Ventilkörper mit seinem Haltebereich in das Verankerungsteil eingeschoben wird oder daß zum Einbringen des Haltebereichs des Ventilkörpers in den Haltebereich des Verankerungsteils nach Einführen des den Ventilkörper enthaltenden Kanülenrohrs bis vor den verjüngten Endabschnitt des Verankerungsteils in ihrem freien Endbereich aufeinander zu gebogene elastische Finger eines den Ventilkörper umgebenden, innerhalb des Kanülenrohrs angeordneten Führungsrohrs aus dem Kanülenrohr heraus und in den verjüngten Endbereich des Verankerungsteils mit dem Ventilkörper eingefahren werden, bis der Haltebereich des Ventilkörpers in den Haltebereich des Verankerungsteils gelangt, und daß anschließend Kanülenrohr und Führungsrohr relativ zum Gegenhalter zurückgezogen werden, wodurch zunächst der Haltebereich des Ventilkörpers innerhalb des Haltebereichs des Verankerungsteils freigegeben wird und anschließen der gesamte Ventilkörper in die Urethra freigegeben wird. Das Einführen der Einführelemente und das Ausbringen des Verankerungsteils und/ oder des Ventilkörpers erfolgt dabei vorzugsweise unter Sicht mittels eines Endoskops.

Weitere Vorteile und Merkmale der Erfindung ergeben sich aus den Ansprüchen und aus der nachfolgenden Beschreibung, in der bevorzugte Ausführungsbeispiele der Erfindung unter Bezugnahme auf die Zeichnungen im einzelnen erläutert sind. Dabei zeigt:
- Fig. 1a: einen schematischen Längsschnitt durch den Urogenitalbereich des Mannes mit einer Darstellung eines in der männlichen Urethra eingesetzten erfindungsgemäßen künstlichen Endosphinkters in seiner Verwendungsposition;
- Fig. 1b: einen Querschnitt entsprechend IB-IB der Fig. 1a durch den Penis;
- Fig. 2: die Darstellung einer ersten Ausgestaltung eines erfindungsgemäßen Endosphinkters, wobei das Ventil lediglich schematisch dargestellt ist;
- Fig. 3: die Darstellung einer weiteren Ausgestaltung des erfindungsgemäßen Endosphinkters mit lösbarem Verankerungsteil und Ventilkörper im zusammengesetzten Benutzungszustand;
- Fig. 4a: eine Darstellung einer ersten äußerst bevorzugten Ausführungsform des Verankerungsteils in seiner radial komprimierten Einführungskonfiguration;
- Fig. 4b: eine Darstellung des Verankerungsteils der Fig. 4a in radial expandierter Benutzungskonfiguration;
- Fig. 5: eine schematische Darstellung einer weiteren Ausführunsform eines erfindungsgemäßen Verankerungsteils;
- Fig. 6a: eine schematische Darstellung einer ersten Ausführungsform des vorderen oder äußeren Endes des Ventilkörper mit dem in diesem befindlichen Ventil im Längsschnitt;
- Fig. 6b: einen Querschnitt entsprechend VI-VI durch die Ausgestaltung des Ventilkörpers mit Ventil der Fig. 6a;
- Fig. 7a: eine Darstellung entsprechend der Fig. 6a mit einer weiteren Ausführungsform eines erfindungsgemäßen Ventils;
- Fig. 7b: eine Darstellung entsprechend der der Fig. 6b zur Ausgestaltung der Fig. 7a;
- Fig. 8: eine weitere Ausgestaltung eines erfindungsgemäßen Ventils im vorderen Teil des Ventilkörpers;
- Fig. 9a: ebenfalls einen Längsschnitt einer anderen Ausgestaltung des erfindungsgemäßen Ventils im vorderen Bereich des Ventilkörpers;
- Fig. 9b: einen Schnitt entsprechend der Fig. 6a für die Ausgestaltung des Ventils der Fig. 9a;
- Fig. 10: eine Darstellung von Einführelementen für einen erfindungsgemäßen Endosphinkter entsprechend der Fig. 2a mit eingebrachtem Endosphinkter;
- Fig. 11: die Darstellung des wesentlichen Verfahrensschritts des Freigebens des Endosphinkters der Fig. 2a aus den Einführelementen der Fig. 10 in die Urethra im Bereich des Beckenbodens;
- Fig. 12: die Darstellung von Einführelementen für das Verankerungsteil der Fig. 4a und 4b eines Endosphinkters der Fig. 3 in der Position der Freigabe des Verankerungsteils in die Urethra im Bereich des Beckenbodens;
- Fig. 13: Einführelemente zum Einführen des Ventilkörpers der Ausgestaltung der Fig. 3 des erfindungsgemäßen Endosphinkters;
- Fig. 14: den wesentlichen Verfahrensschritt zum Einbringen des Ventilkörpers mittels der Einführelemente 81 in das vorher eingesetzte Verankerungsteil der Ausgestaltung der Fig. 3, 4a und 4b;
- Fig. 15a: eine teilweise Längsschnittdarstellung einer weiteren Ausführungsform von Einführelementen zur Einführung des Ventilkörpers der Ausgestaltung der Fig. 3 des erfindungsgemäßen Sphinkters in das vorher gelegte Verankerungsteil;
- Fig. 15b: eine Stirnansicht des Gegenstandes der Fig. 15a vor Ausbringen des Ventilkörpers;

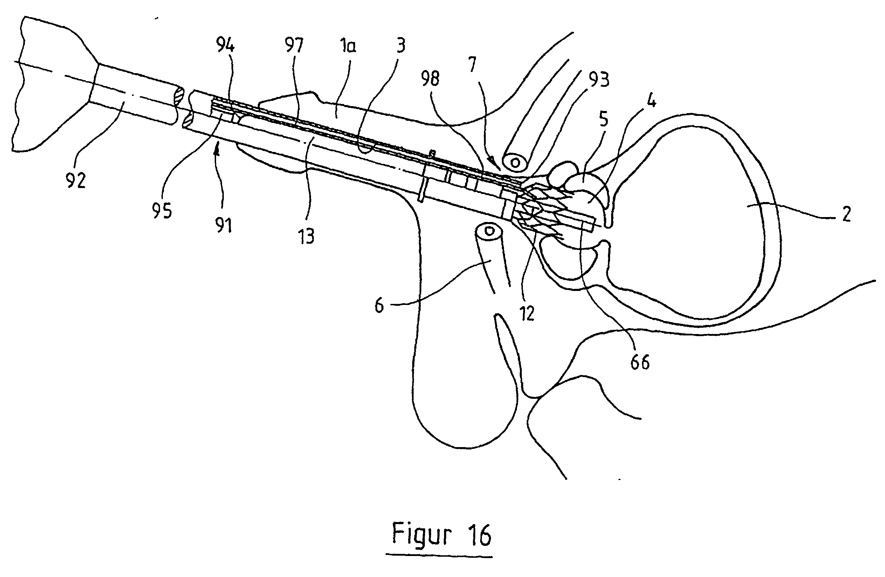

- Fig. 16: den wesentlichen Verfahrensschritt zum Einführen des Verankerungsbereichs des Ventilkörpers in ein vorher gelegtes Verankerungsteil.

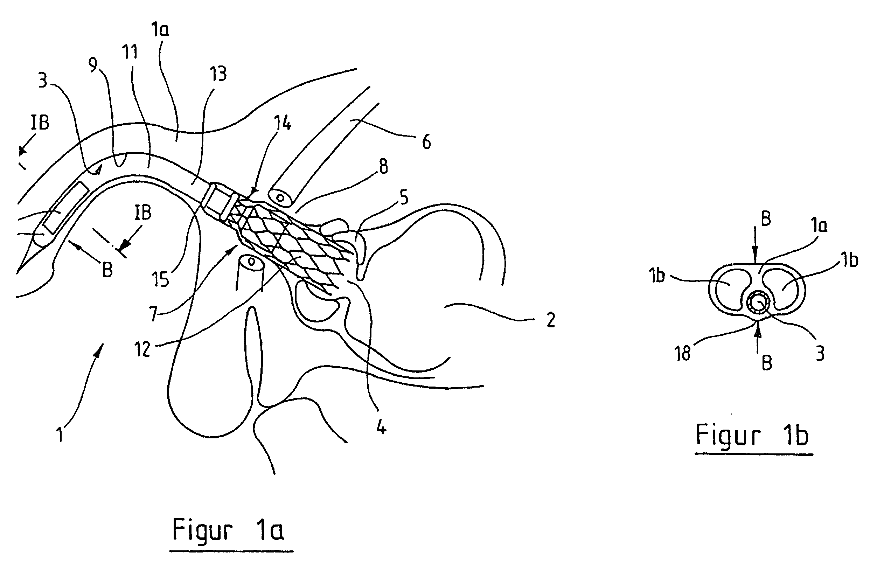

Die Fig. 1a zeigt eine schematische Darstellung des Urogenitalbereichs 1 des Mannes, wobei ein erfindungsgemäßer Endosphinkter durch die äußere Urethraöffnung in die männliche Urethra eingebracht ist.

Aus der Blase 2 tritt die Urethra 3 im Penis 1a mit der prostatischen Urethra 4 mit einer Länge von ca. 3 bis 4 cm aus. Es ist ersichtlich, daß zumindest ein Teil der Prostata 5 entfernt wurde. Mit 6 ist das Diaphragma urogenitale, das Teil des Beckenbodens ist, bezeichnet, der durch die Pars membranacea 7 mit einer Länge von etwa 1 bis 2 cm durchbohrt ist, welche wiederum üblicherweise durch den äußeren Sphinkter 8 (musculus sphincter urethrae) umgeben ist. An die Pars membranacea 7 der Urethra 3 schließt sich ihre Pars spongiosa 9 im Corpus spongiosum an, die schließlich in der äußeren Urethraöffnung 10 mündet (Ostium urethrae externum). Die Urethra 3 liegt im unteren Bereich des Penis 1a unterhalb und zwischen den Schwellkörpern 1b.

Bei dem Patienten, dessen Urogenitalbereich 1 in der Fig. 1a dargestellt ist, mußte aufgrund einer Entfernung oder Lädierung des äußeren Sphinkters 8 oder einer Hyperaktivität desselben ein erfindungsgemäßer künstlicher Endosphinkter 11 eingesetzt werden, wie er in seiner Gebrauchsposition in der Fig. 1a dargestellt ist.

Der Endosphinkter 11 weist ein Verankerungsteil 12 auf, welches einen Ventilkörper 13 hält. Das Verankerungsteil 12 ist innerhalb der Stelle des äußeren Sphinkters 8, also im Bereich der Pars membranacea 7 angeordnet. Die genaue Position hängt dabei von der jeweiligen Situation ab. Vom Verankerungsteil 12 erstreckt sich der Ventilkörper 13 in die Pars spongiosa 9.

Der Ventilkörper 13 weist nahe seines inneren, durch das Verankerungsteil 12 gehaltenen Endes 14 Dichtungslippen 15 auf, wobei im dargestellten Ausführungsbeispiel zwei Dichtungslippen vorhanden sind, die den Ventilkörper 13 radial überragen. Der Ventilkörper 13 ist schlauchartig hohl ausgebildet mit einer Mantelwand 13a. Im äußeren, dem Verankerungsteil 12 abgewandten Endbereich 16 ist ein Ventil 17 angeordnet, das in verschiedenartiger Weise ausgebildet sein kann und weiter unten im einzelnen erläutert wird. Der Endosphinkter 11 ist in der Urethra 3 derart angeordnet, daß das Ventil 17 durch Druckausübung von der Unterseite des Penis 1a (im Bereich der durch Verwachsen der Geschlechtsfalten gebildeten Penisnaht 18) und von der Oberseite des Penis 1a in Richtung der Pfeile B und B' geöffnet werden kann.

Solange kein Druck auf das Ventil 17 ausgeübt wird, ist das Ventil geschlossen. Der Urin wird in der Harnblase 2 und im Ventilkörper 13 zurückgehalten; der Patient ist daher durch den erfindungsgemäßen Endosphinkter 11 kontinent, kann aber durch die beschriebene Druckausübung auf das Ventil 17 dieses öffnen und damit Harn ablassen.

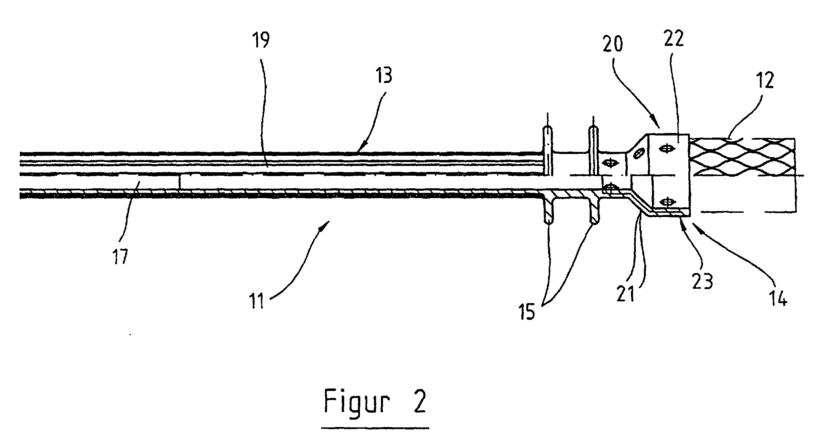

Die Fig. 2 zeigt eine erste Ausführungsform des erfindungsgemäßen Endosphinkters 11. Bei dieser Ausgestaltung sind Ventilkörper 13 und Verankerungsteil 12 derart fest miteinander verbunden, daß sie nicht ohne Beschädigung voneinander trennbar sind. Der Ventilkörper 13 weist einen zylindrischen Hauptkörper 19 auf, in dessen vorderem oder äußerem Endbereich 16, wie gesagt, das Ventil 17 eingelassen ist. Am Außenumfang einer Mantelwandung 13a des Ventilkörpers 13 sind Längsrillen 13b zwischen Längsrippen 13c ausgebildet. Die Längsrillen dienen als Drainagerillen zum Ableiten von sich in der Urethra bildendem Sekret zum Harnleiterausgang. Der zylindrische Hauptkörper 19 erweitert sich in einen Haltebereich 20 über eine Schulter 21 zu einem ebenfalls zylindrischen Endabschnitt 22. Der Hauptkörper 19 hat damit einen ersten Durchmesser, der geringer ist als der Durchmesser des Endabschnitts 22. In auf einem dem Endabschnitt 22 nahen Bereich des Hauptkörpers 19 ist dieser durch die elastischen Dichtlippen 15 umgeben. Der Durchmesser der elastischen Dichtlippen 15 liegt in der Größenordnung des Endabschnitts 22, vorzugsweise geringfügig über dem Durchmesser des Endabschnitts 22. Bei der Ausgestaltung der Fig. 2 ist in den Endabschnitt 22, die Schulter 21 und einen kurzen Teil des Hauptkörpers 19 ein Haltebereich 23 des Verankerungsteils 12 eingelassen bzw. eingegossen. Der Ventilkörper 13 besteht vorzugsweise aus Silikon oder relativ weichem Kunststoffmaterial, das Verankerungsteil 12 aus einer durchbrochenen Struktur aus Metall, und zwar insbesondere aus einer Formgedächtnislegierung, wie einer Nickel-Titan-Legierung; eine solche ist unter der Bezeichnung Nitinol bekannt. In diesem Falle hat das Verankerungsteil bei relativ tiefen Temperaturen, insbesondere Temperaturen deutlich unterhalb der Körpertemperatur von 37 °C, eine Kontur mit einem geringen Durchmesser und weitet sich erst oberhalb einer Übergangstemperatur, die ebenfalls unterhalb der Körpertemperatur liegt, in seine Hochtemperaturkontur mit relativ großem Durchmesser auf, wie sie in den Fig. 1 und 2 dargestellt ist.

Der erfindungsgemäße Endosphinkter 11 der Fig. 2, bei dem also Verankerungsteil 12 und Ventilkörper 13 fest miteinander verbunden sind, wird als solcher insgesamt durch die Harnleiteröffnung 10 in die Urethra 3 eingeführt.

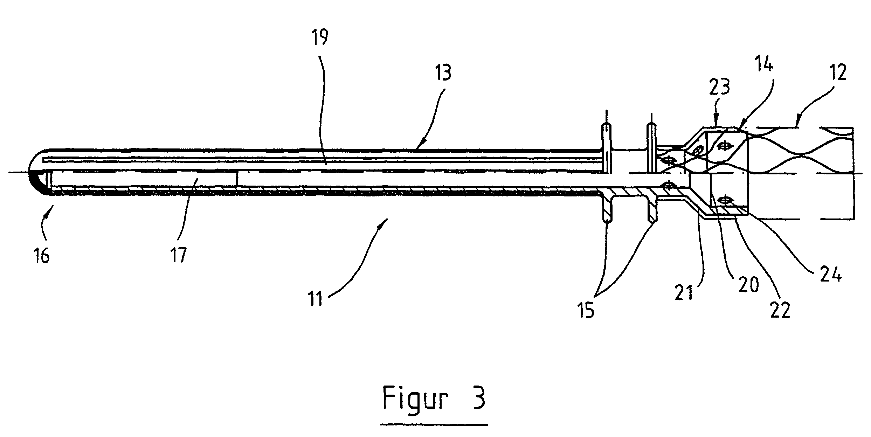

Die Fig. 3 zeigt eine weitere Ausgestaltung des erfindungsgemäßen Endosphinkters 11 in seinem Einsatzzustand. Gleiche Teile sind mit gleichen Bezugszeichen bezeichnet.

Bei der Ausgestaltung der Fig. 3 sind der Ventilkörper 13 und das Verankerungsteil 12 lösbar miteinander verbunden, indem der Haltebereich 20 oder Kopf des Ventilkörpers 13 formschlüssig innerhalb des Haltebereichs 23 des Verankerungsteil 12 gehalten ist. Da sowohl das Verankerungsteil 12 als auch der Ventilkörper 13 und insbesondere der Haltebereich 20 flexibel-elastisch ausgebildet sind, kann der Ventilkörper 13 gegebenenfalls vom Verankerungsteil 12 getrennt und entfernt werden, wodurch ein einfacher Austausch alleine des Ventilkörpers 13 möglich ist, wenn dieser beispielsweise inkrustiert ist, während das Verankerungsteil 12 an seiner in der Fig. 1 dargestellten Position in der Urethra membranacea verbleibt. Dies ist äußerst vorteilhaft, da das Verankerungsteil 12 durch Epithelgewebe überwachsen ist.

Der Ventilkörper 13 weist insbesondere im Endbereich 22 sowie in seinem Bereich zwischen diesem und der ersten Dichtlippe Drainageöffnungen 24 auf.

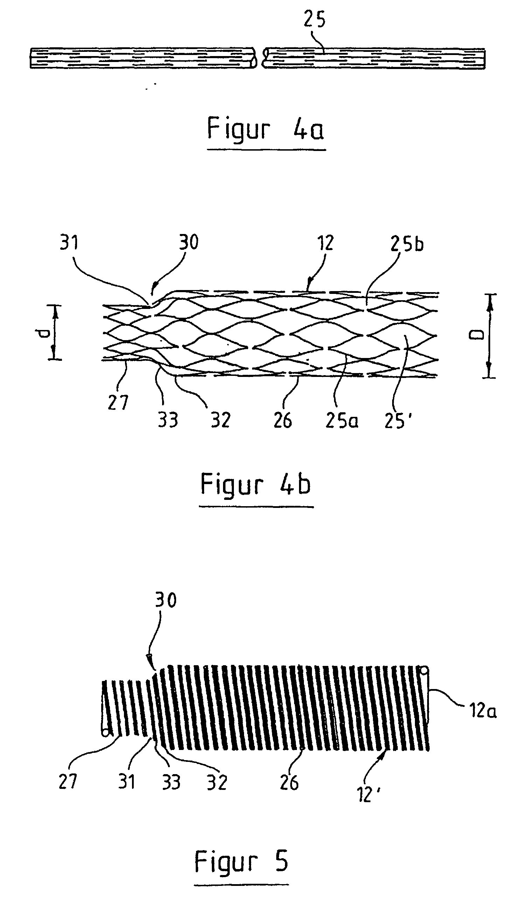

Eine äußerst bevorzugte Ausgestaltung eines Verankerungsteils 12 des erfindungsgemäßen Endosphinkters 11 ist in den Fig. 4a und b dargestellt. Die Fig. 4a zeigt dabei die Einführ- oder Tieftemperaturkonfiguration des Verankerungsteils 12 mit sehr geringem Durchmesser, während die Fig. 4b die Einsatzkontur oder Hochtemperaturkontur zeigt. Das Verankerungsteil 12 besteht aus einem sehr dünnen Blech mit einer Vielzahl in seiner Haupterstrecküngsrichtung hintereinander angeordneter und über den Umfang versetzt angeordneter Schlitze oder Einschnitte 25. In der Hochtemperaturposition weiten sich diese Einschnitte zu rautenförmigen Durchbrechungen 25'.

Die Schlitze 25 bzw. die Durchbrechungen 25' begrenzende Stege oder Rippen 25a sind teilweise aufgeschnitten (Schnitte 25b); hierdurch wird eine hohe angulare Biegeflexibilität des Verankerungsteils 12 erzielt.

Die Außenkontur des Verankerungsteils 12 in der in Fig. 4b dargestellten Hochtemperaturstellung ist die folgende: Das Verankerungsteil 12 weist einen zylindrischen Hauptkörper 26 mit einem ersten Durchmesser auf, der im wesentlichen dem Durchmesser des Endabschnitts 22 des Ventilkörpers 13 entspricht. Das Verankerungsteil 12 weist weiterhin einen ebenfalls zylindrischen Endabschnitt 27 auf, der einen kleineren Durchmesser hat als der Hauptkörper 26. Zwischen dem Abschnitt 27 und dem Hauptkörper 26 ist der sich von ersterem zu letzterem hin erweiternde Haltebereich 30 vorgesehen.

Der Haltebereich 30 ist sich schulterartig erweiternd ausgebildet. Er weist ausgehend vom Endabschnitt 27 zunächst einen ersten sich trompetenartig erweiternden Erweiterungsabschnitt 31 und weiterhin einen sich vom Hauptkörper 26 zum Erweiterungsabschnitt 31 hin bogenförmig eingezogenen oder verjüngenden Verjüngungsabschnitt 32 auf. Im dargestellten Ausführungsbeispiel ist zwischen den Abschnitten 31 und 32 weiterhin ein sich teilkonisch erweiternder Zwischenabschnitt 33 ausgebildet.

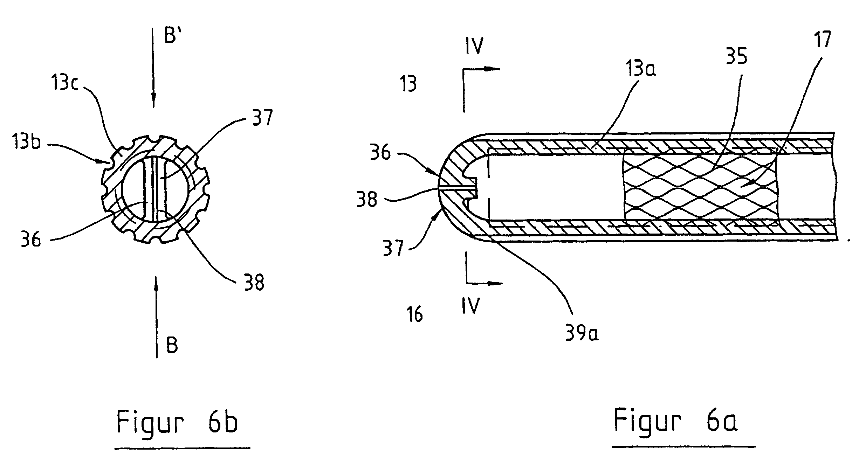

Die Fig. 5 zeigt eine weitere Ausgestaltung eines erfindungsgemäßen Verankerungsteils 12', das durch einen schraubenförmig gewundenen Draht gebildet ist und bei dem grundsätzlich die gleichen Abschnitte vorgesehen sind wie bei der Ausgestaltung der Fig. 4a und 4b. Auch der Draht 12a des Verankerungsteils 12' besteht aus einer Formgedächtnislegierung (Memory-Metall-Legierung) der schon erläuterten Art und hat ebenfalls eine Tieftemperaturstellung mit sehr geringem Durchmesser.

Die Fig. 6a und 6b zeigen nun eine erste Ausgestaltung des Ventilkörpers 13 des erfindungsgemäßen Endosphinkters 11.

Bei der Ausgestaltung der Fig. 6a und 6b weist das Ventil 17 ein flexibel-elastisches Zylindermantelteil 35 auf, das im Mantel 13a des Ventilkörpers 13 eingelassen ist. Zur Veranschaulichung wurde teilweise ein innerer Wandungsbereich des Mantels 13a des Ventilkörpers 13 weggebrochen, so daß das eingelassene Zylindermantelteil 35 dort deutlich erkennbar ist. Es kann ebenfalls vorzugsweise mit Durchbrechungen, wie rautenartigen Durchbrechungen ähnlich denen bei der Ausgestaltung der Fig. 4b des Verankerungsteils 12 ausgebildet sein. Das Zylindermantelteil 35 besteht vorzugsweise aus korrosionsfreiem Edelstahl.

Der äußere Endbereich 16 des Ventilkörpers 13 ist stirnseitig abgerundet und mit zwei vertikal gerichteten Dichtlippen 36, 37 versehen, die zwischen sich eine Schlitzöffnung 38 einschließen. Die Dichtlippen 36, 37 sind zum Inneren des Ventilkörpers 13 hin mit Ansätzen 39, 39a versehen. Im unbelasteten Zustand schließen die Dichtlippen 36, 37 die hier gestreckt ausgebildete Schlitzöffnung 38 dicht ab, so daß keine Flüssigkeit aus dem Inneren des Ventilkörpers 13 austreten kann. Bei Druckausübung in Richtung der Pfeile B, B', also senkrecht zur Erstreckungsrichtung des Schlitzes 38 auf den äußeren Endbereich 16 des Zylinderkörpers 35, werden die Dichtlippen 36, 37 auseinandergedrückt, so daß die Schlitzöffnung 38 sich öffnet und Flüssigkeit aus dem Inneren des Ventilkörpers 13 in die Harnröhre und aus deren äußerer Öffnung heraustreten kann.

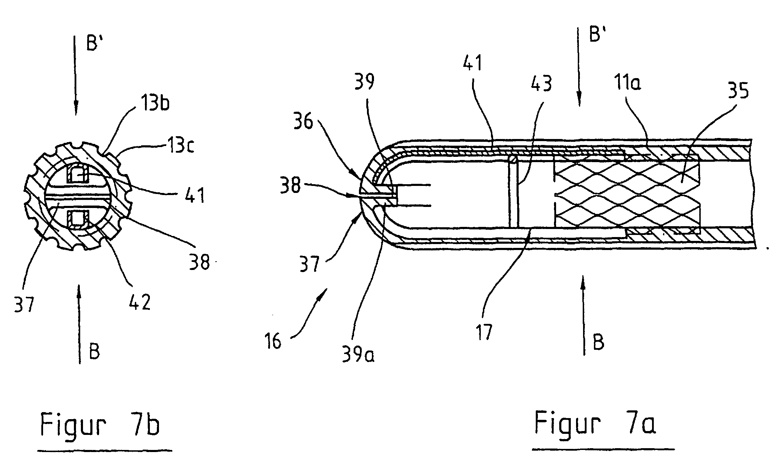

Bei der Ausgestaltung der Fig. 7a und 7b ist ebenfalls ein elastischer, zylindrischer Körper 35 vorgesehen. Dieser ist aber vom vorderen Ende 16 des Ventilkörpers mit größerem Abstand angeordnet. Er ist auf der Ober- und Unterseite mit Hebeln 41, 42 versehen, wobei der Hebel 41 sich am Inneren der Wandung 13a des Ventilkörpers 13 entlang bis über den Ansatz 39 der Dichtlippe 36 erstreckt, während der Hebel sich in gleicher Weise bis unterhalb des Ansatzes 39 der Dichtlippe 37 erstreckt. Zwischen den Ansätzen 39, 39a und dem elastischen Körper 35 ist ein Scharnierteil 43 angeordnet, das fest mit den Hebeln 41, 42 verbunden, selbst aber elastisch ist.

Durch diese Ausgestaltung wird bei Druckausübung in Richtung der Pfeile B, B' auf die auf der Außenseite des elastischen Körpers 35 liegenden Enden der Hebel 41, 42 durch Anheben der im Ende 16 des Ventilkörpers 13 befindlichen gegenüberliegenden Enden der Schlitz 38 geöffnet; das Scharnierteil 43 biegt sich dabei leicht. Es kann eine größere Hebelwirkung gegenüber der Ausgestaltung der Fig. 6a, 6b erreicht werden. Die Druckrichtung relativ zur Erstreckungsrichtung der Schlitzöffnung 38 ist unterschiedlich, hier senkrecht zur Erstreckungsrichtung, während bei der Ausgestaltung der Fig. 6a, 6b die Druckausübung in Erstreckungsrichtung der Schlitzöffnung 38 erfolgte; bezogen auf den menschlichen Körper bzw. den Penis muß aber immer, wie gesagt, die Druckausübung von oben und unten erfolgen.

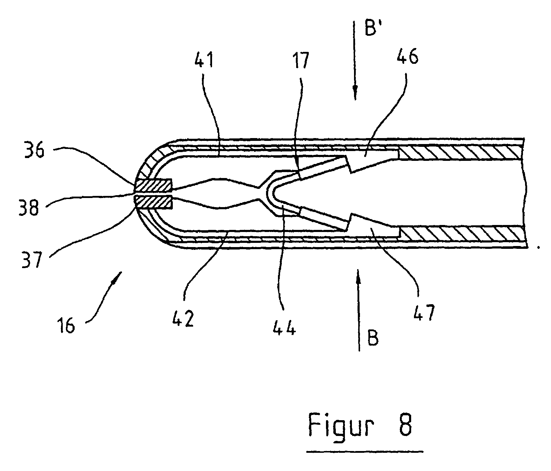

Eine weitere Ausgestaltung eines erfindungsgemäßen Ventils ist in der Fig. 8 dargestellt. Diese Ausgestaltung arbeitet ebenfalls mit (zweiarmigen) Hebeln 41, 42 entsprechend der Ausgestaltung der Fig. 7a und b. Das elastische Rückstellteil, das bei der Ausgestaltung der Fig. 6a, 6b, 7a, 7b ein zylinderförmiger, elastischer Körper war, ist hier durch eine Bügelfeder 44 ersetzt, die im Bereich der innenseitigen Enden 46, 47 der Hebel 41, 42 angreift und sich V-förmig zur Stirnseite 16 hin bis etwa zur Mitte der Länge der Hebel 41, 42 erstreckt. Diese greifen auch wieder ober- und unterseitig von Dichtlippen 36, 37 an, die hier nicht einstückig mit der Wandung 13a oder dem Mantel des Ventilkörpers 13 ausgebildet, sondern als separate Teile im vorderen Ende 16 eingelassen sind. Die Betätigung des Ventils erfolgt ebenfalls wieder in Richtung der Pfeile B, B' im Bereich der Enden 46, 47 der Hebel 41, 42, die so als Betätigungsflächen dienen.

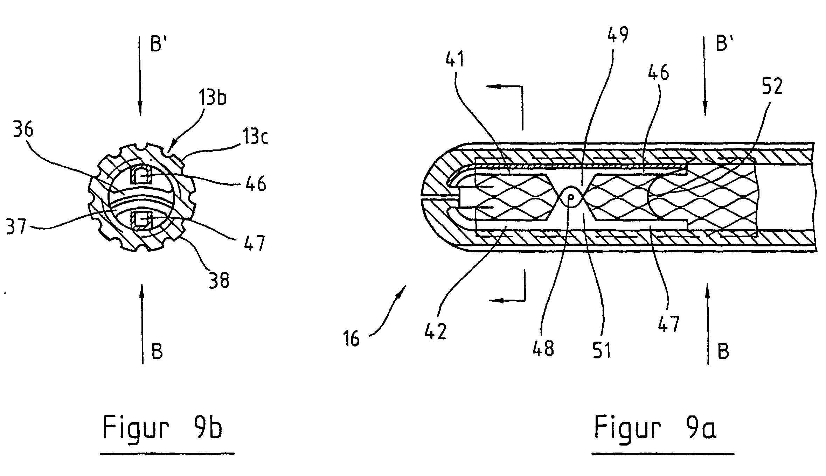

Bei der Ausgestaltung der Fig. 9a, 9b sind ebenfalls wieder zweiarmige Hebel 41, 42 vorgesehen. Diese weisen ein Drehgelenk 48 an zwei von den Hebeln 41, 42 aufeinander zu gerichteten Gelenklagerteilen 49, 51 auf. Als Rückstellglied ist eine Bügelfeder 52 zwischen den der Stirnseite 16 abgewandten Enden 46, 47 der Hebel 41, 42 angeordnet. Zusätzlich kann, wie dargestellt, auch zur Aussteifung des zylindrischen Hauptkörpers 19 des Ventilkörpers 13 in der Wandung 13a ein flexibel elastisches Zylindermantelteil 35, wie bei den Ausgestaltungen der Figuren 6a, 6b, 7a, 7b, eingelassen sein.

Die Schlitzöffnung 38 ist hier, wie aus der Fig. 9b ersichtlich ist, bogenförmig ausgebildet.

Bei Druck in Richtung der Pfeile B, B' im Bereich der Enden 46, 47 verschwenken die Hebel 41, 42 um das Drehgelenk 48 und öffnen auch hier die Schlitzöffnung 38.

Zu den unter Bezugnahme auf die Fig. 6a bis 9b erläuterten Ausgestaltungen des Ventils ist zu sagen, daß einzelne Elemente einer Ausgestaltung auch mit anderen Elementen einer anderen Ausgestaltung kombiniert werden können, so kann beispielsweise auch die gebogene Schlitzöffnung 38 der Ausgestaltung der Fig. 9a und 9b bei den Ventilausbildungen der anderen erläuterten Figuren eingesetzt werden und bei der Ausgestaltung der Fig. 9a auch ein gerader Schlitz entsprechend der Fig. 6b und 7b.

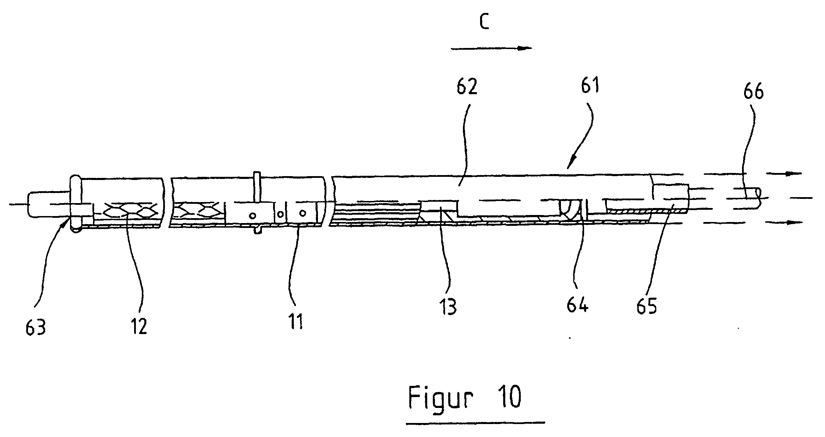

Die Fig. 10 zeigt in die Urethra einführbare Einführelemente 61 für einen erfindungsgemäßen Endosphinkter 11 in der Ausgestaltung der Fig. 2, bei der Verankerungsteil 12 und Ventilkörper 13 in der dort gezeigten Weise fest miteinander verbunden sind. Die Einführelemente 61 weisen zunächst ein äußeres Kanülenrohr 62 auf, in dem der Endosphinkter 11 mit Ventilkörper 13 und Verankerungsteil 12 unter radialer Kontraktion aufgenommen ist. Das Verankerungsteil 12 ist dabei nahe der Austrittsöffnung 63 des Kanülenrohrs 62 angeordnet. Am dem Verankerungsteil 12 abgewandten Ende 22 des Ventilkörpers 13 liegt der Anschlag 64 eines zweiten, inneren Kanülenrohres 65 an. Durch die gesamte Anordnung und daher auch durch das Kanülenrohr 62 erstreckt sich eine optische Faser 66 eines Endoskops, wobei hierzu das Ventil (nicht dargestellt) des Ventilkörpers 13 eben leicht geöffnet ist.

Das äußere Kanülenrohr 62 und das innere Kanülenrohr 65 sind relativ zueinander bewegbar, genauer ist das äußere Kanülenrohr 62 relativ zum inneren Kanülenrohr 65 in Richtung des Pfeiles C zurückziehbar. Hierzu können die Kanülenrohre 62, 65 an ihrem der Ausbringöffnung 63 abgewandten Ende mit Betätigungsvorrichtungen zum Relativbewegen der Kanülenrohre 62, 65 versehen sein, wie sie beispielsweise in der DE 44 20 142 A1 beschrieben sind.

Das Einführen eines erfindungsgemäßen Endosphinkters der Ausgestaltung der Fig. 2 erfolgt derart, daß zunächst die Einführelemente 61 mit im Kanülenrohr 62 aufgenommenem Endosphinkter entsprechend der Ausgestaltung der Fig. 10 durch die Öffnung 10 der Harnröhre 3 in diese eingeführt werden. Die Ausbringöffnung 63 des Kanülenrohrs 62 wird dabei bis über das Diaphragma genitale bzw. den Beckenboden 6 hinausgeschoben. Bei diesem Einführen der Einführelemente 61 verändern die Kanülenrohre 62, 65 ihre relative axiale Lage nicht. Die genaue Positionierung kann über die optische Faser 66 des Endoskops beobachtet werden.

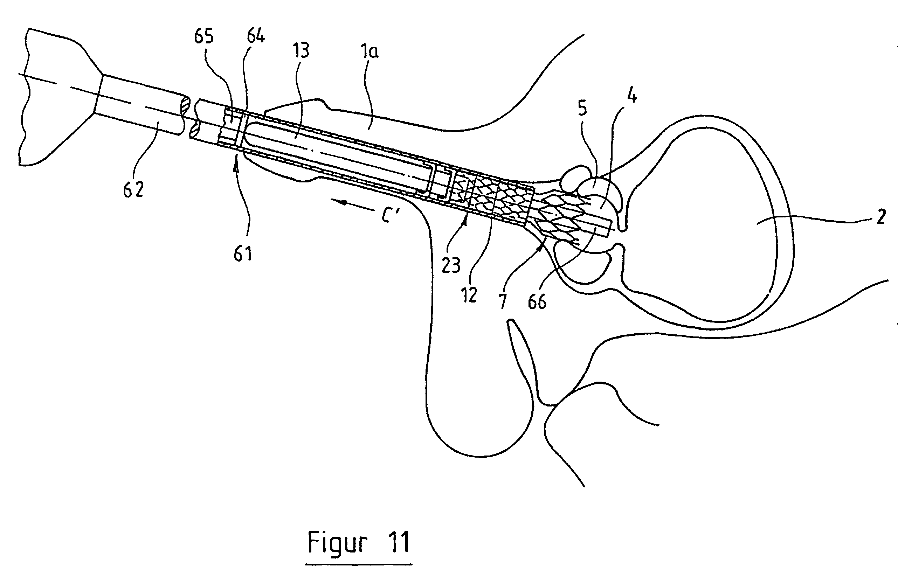

Anschließend wird das äußere Kanülenrohr 62 in Richtung des Pfeiles C (Fig. 10 und 11) zurückgezogen, und zwar auch relativ zum inneren Kanülenrohr 65, welches mit seinem Anschlag 64 als Gegenhalter an dem Ende 16 des Endosphinkters 11 anliegt. Hierdurch wird zunächst das Verankerungsteil 12 im Bereich des Beckenbodens 6 aus der Ausbringöffnung 63 des äußeren Kanülenrohrs 62 freigegeben, wie dies in der Fig. 11 dargestellt ist. Beim weiteren Zurückziehen des äußeren Kanülenrohrs 62 in Richtung des Pfeiles C wird nach und nach der gesamte Endosphinkter und insbesondere auch sein Ventilkörper 13 freigegeben, bis auch der äußere Endbereich 16 des Ventilkörpers 13 aus dem äußeren Kanülenrohr 62 freigegeben ist. Anschließend können die Einführelemente 61 durch die Urethraöffnung 10 aus der Urethra entfernt werden.

Der erfindungsgemäße Endosphinkter 11 der Ausgestaltung der Fig. 2 ist derart plaziert und kann in der beschriebenen Weise benutzt werden. Soweit sein Ventil unbelastet ist, hält er den Urin zurück; durch Belastung, d.h. Druckausübung in Richtung der Pfeile B, B', kann in der beschriebenen Weise die Blase des Patienten entleert werden.

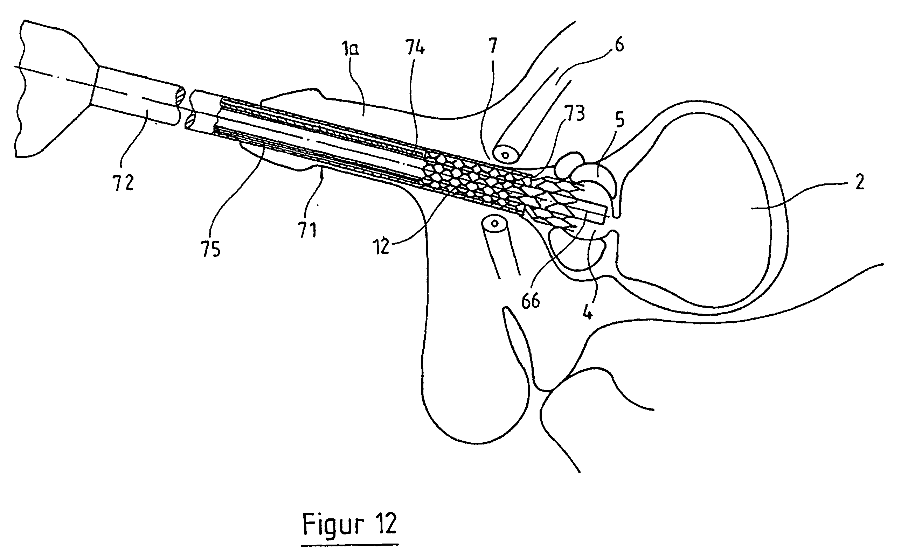

Die Fig. 12 zeigt die wesentlichen Einführelemente 71 eines Einführbestecks zum Einführen zunächst lediglich des Verankerungsteils 12 der Ausgestaltung der Fig. 3 des erfindungsgemäßen Endosphinkters 11, bei denen Verankerungsteil 12 und Ventilkörper 13 lösbar miteinander verbindbar sind. Auch hier ist zunächst ein äußeres Kanülenrohr 72 und ein als Anschlag oder Gegenhalter dienendes inneres Kanülenrohr 75 vorgesehen. Die Ausbringöffnung des äußeren Kanülenrohres 72 ist mit 73 bezeichnet. Das innere Kanülenrohr 75 liegt mit seinem Ende 74 an dem nach außen gerichteten Ende des Verankerungsteils 12 an. Durch die gesamte Anordnung erstreckt sich auch hier wieder die Faser 76 eines nicht näher dargestellten Endoskops. Die Einführelemente 71 sind an ihrem der Ausbringöffnung 73 abgewandten Ende ebenfalls durch einen Mechanismus zu Relativbewegungen derselben verbunden. Zum Einführen der Einführelemente 71 ist das Verankerungsteil 12 zunächst voll im äußeren Kanülenrohr 72 aufgenommen. Die Einführelemente 71 werden in der vorstehend unter Bezugnahme auf die Fig. 11 beschriebenen Weise in die Harnröhre 3 eingeführt, bis das Ausbringende 73 des äußeren Kanülenrohrs 72 den Beckenboden 6 durchschritten hat. Anschließend wird das äußere Kanülenrohr 72 unter Gegenhaltung des inneren Kanülenrohrs 75 relativ zu diesem in Richtung des Pfeiles C zurückgezogen, wodurch das Verankerungsteil 12 freigegeben wird und sich in der Harnröhre 3, insbesondere in deren Pars membranacea 7, positionieren kann. Die Einführelemente werden schließlich vollständig aus der Urethra entfernt.

In der Regel wird zunächst eine Epithelbildung auf dem Verankerungsteil 12 abgewartet, bevor dann auch der Ventilkörper 13 eingebracht wird.

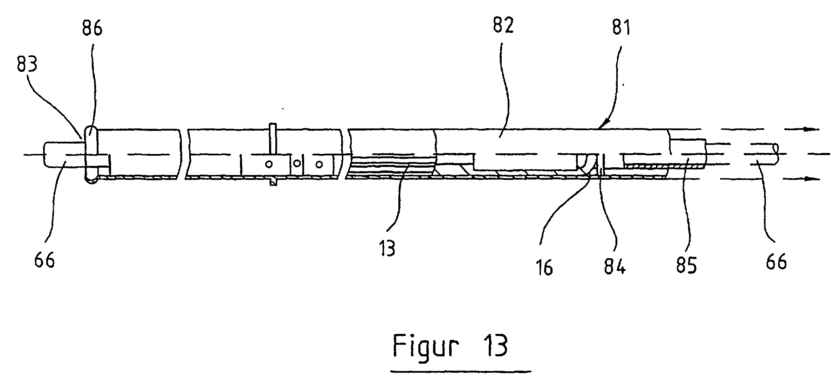

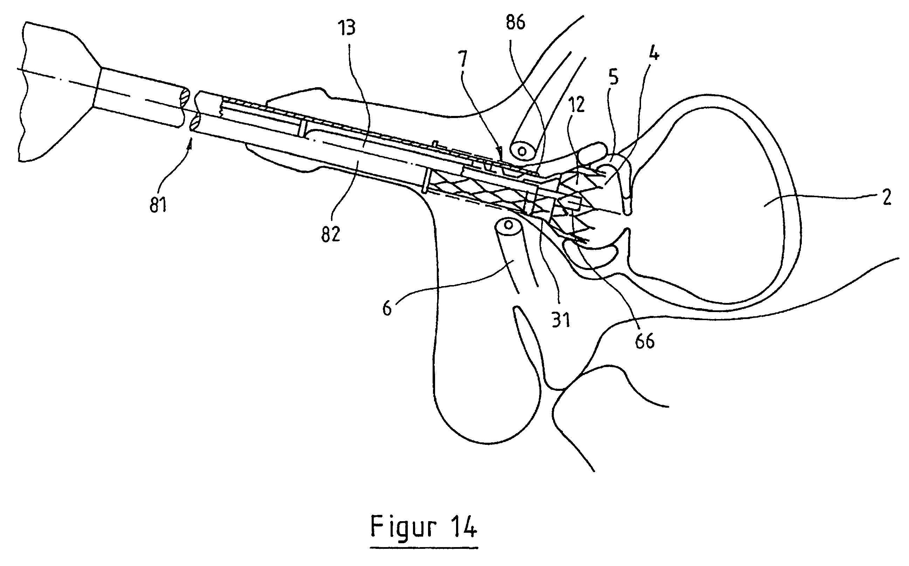

Die Fig. 13 zeigt zunächst eine erste Ausgestaltung der wesentlichen Einführelemente 81 für einen Ventilkörper 13 nach der Fig. 3. Die Ausgestaltung der Einführelemente 81 ist im wesentlichen ähnlich der der in Fig. 10 beschriebenen Einführelemente 61. Es ist ein äußeres Kanülenrohr 82 mit einer Ausbringöffnung 83 vorhanden. In dem äußeren Kanülenrohr 82 befindet sich als Gegenhalter relativ zu diesem bewegbar ein inneres Kanülenrohr 85 mit einem am äußeren Endbereich 16 des Ventilkörpers 13 anliegenden Anschlag 84. Durch die gesamte Anordnung erstreckt sich wieder eine Faseroptik 66. Das äußere Kanülenrohr 82 weist seine Ausbringöffnung 83 umgebend einen abgerundeten Einführabschnitt 86 auf. Der zum freien Ende hin etwas bogenförmig verjüngte Durchmesser des Einführabschnitts 86 liegt unterhalb des inneren Durchmessers des Verankerungselements 12 in seinem verjüngten zylindrischen Endbereich 27 im entlasteten Zustand des Verankerungselements 12, wenn dieses also seine aufgeweitete oder Hochtemperaturkontur eingenommen hat, wie sie in der Fig. 4b, aber auch in der Fig. 14 dargestellt ist.

Zum Einführen der Einführelemente 81 in die Harnröhre 3 eines Patienten befindet sich der Ventilkörper 13 wieder vollständig in dem äußeren Kanülenrohr 82, wie dies in der Fig. 13 dargestellt ist. Das Einführen der Einführelemente 81 in die Urethra 3 erfolgt grundsätzlich ebenfalls in der oben beschriebenen Weise.

Wenn das Ende 86 des äußeren Kanülenrohrs 82 vor das Verankerungsteil 12 gelangt, wird es unter Sicht in das verjüngte zylindrische Ende 27 des Verankerungsteil 12 eingeführt, was aufgrund der beschriebenen Durchmesserverhältnisse möglich ist. Das äußere Kanülenrohr 82 wird mit seinem Ende 86 bis über den Bereich 32 des Verankerungsteils 12 hinausgeführt. Bei dem gesamten Einführen bleibt die Relativposition von äußerem Kanülenrohr 82 und innerem Kanülenrohr 85 wieder fest. Anschließend wird das äußere Kanülenrohr 82 relativ zum inneren Kanülenrohr 85 wieder zurückgezogen, wodurch zunächst der erweiterte Haltebereich 23 des Ventilkörpers 13 freigegeben wird, so daß er sich im Bereich 32 des Verankerungsteils 12 anlegen kann.

Anschließend wird das äußere Kanülenrohr 82 sukzessive weiter zurückgezogen, wodurch der gesamte Ventilkörper 13 freigegeben wird, bis er völlig frei innerhalb der Urethra 3 liegt und durch das Verankerungsteil 12 gehalten wird. Die Einführelemente werden aus der Urethra entfernt. Das Ventil 17 des Ventilkörpers 13 kann dann in der schon beschriebenen Weise zur Steuerung des Harnflusses benutzt werden.

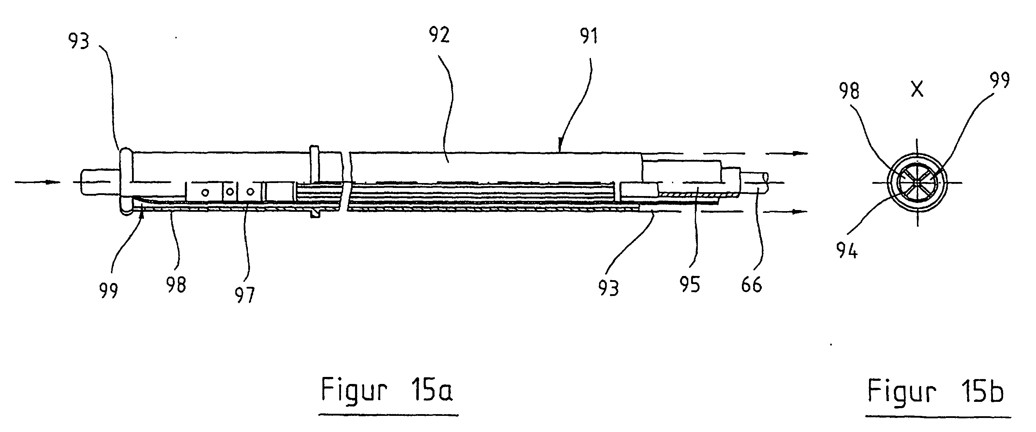

Die Fig. 15 zeigt die Einführelemente 91 einer abgewandelten Ausführungsform eines Einführungsbestecks. Der Durchmesser des äußeren Kanülenrohres 92 ist hier größer als der Durchmesser des Teils 27 des Verankerungsteils 12. Um dennoch ein Einführen des Endes 22, 23 des Ventilkörpers 13 in das Verankerungsteil 12 zu ermöglichen, dessen Ende 27 ja einen geringeren Durchmesser hat als die Teile 22, 23 des Ventilkörpers 13 im radial entlasteten Zustand, weisen die Einführelemente 91 noch zwischen der Wandung des äußeren Kanülenrohrs 92 und dem Ventilkörper 13 ein Führungsrohr 97 auf. Das Führungsrohr 97 ist in seinem vorderen, der Abgabeöffnung 93 zugewandten Ende über eine hinreichende Länge hin in vier Finger 98 gespalten. Die Finger 98 und damit das Führungsrohr 97 verjüngt sich zur Öffnung 93 hin zu einem stumpfen Endbereich 99. Die Finger 98 sind entlastet und können, wenn sie nicht von außen radial zusammengehalten sind (wie hier zunächst durch das Kanülenrohr 92), durch Kraftbeaufschlagung von innen her (durch den Ventilkörper 13 in noch zu beschreibender Weise) radial nach außen gebogen werden und eine Abgabeöffnung freigeben.

Die weiteren Teile der Einführelemente 91 sind bei dieser Ausgestaltung die gleichen wie die bei der Ausgestaltung der Fig. 13. Äußeres Kanülenrohr 92 und inneres Kanülenrohr 95 sind axial relativ zueinander bewegbar. Weiterhin ist das Führungsrohr 97 relativ zu den beiden Kanülenrohren 92, 95 bewegbar.

Das Einführen der Einführelemente 91 in die Urethra 3 geschieht in der schon unter Bezugnahme auf die anderen Ausgestaltungen der Einführelemente beschriebenen Weise unter Sicht mittels des erwähnten Endoskops. Wenn das Abgabeende 93 der Kanüle 92 vor den Bereich 27 des Verankerungsteils 12 gelangt ist, wird das Führungsrohr 97 mit seinen Fingern 98 aus der äußeren Kanüle 92 ausgefahren, wobei die Finger 98 in das Ende 27 des Verankerungsteils einfahren, während das vordere Auslaßende 93 der äußeren Kanüle 92 im Bereich des Endes 27 des Verankerungsteils 12 gehalten wird. Das Einführen des Führungsrohrs 97 mit seinen Fingern 98 erfolgt unter Mitführung des Endosphinkters 11 so weit, bis dessen erweiterter Bereich 22, 23 hinter der Einziehung 32 des Verankerungsteils 12 liegt. Anschließend wird das Führungsrohr 97 zusammen mit dem äußeren Kanülenrohr 92 unter Gegenhaltung des Ventilkörpers 13 mittels des Anschlags 94 des inneren Kanülenrohrs 95 in Richtung des Pfeiles C zurückgezogen. Die Finger 98 des Führungsrohrs 97 sind in dieser Position vom äußeren Kanülenrohr 92 freigegeben und können sich unter dem durch den Ventilkörper 13 ausgeübten Druck radial nach außen aufdehnen, wodurch der Ventilkörper 13, zunächst mit seinem Ende 22, hinter dem Bereich 32 des Verankerungsteils 12 abgelegt wird. Äußere Kanüle 92 und Führungsrohr 97 werden so weit zurückgezogen, bis der gesamte Ventilkörper 13 aus ihnen entfernt ist, frei in der Harnröhre 3 liegt und lediglich in der weiter oben beschriebenen Weise durch das Verankerungsteil 12 gehalten wird. Die Einführelemente werden anschließend aus der Urethra entfernt.

Der erfindungsgemäße Endosphinkter kann dann auch wieder in der beschriebenen Weise benutzt werden.

## Patentansprüche

1. Künstlicher Endosphinkter für die Urethra, mit einem Verankerungsteil (12) und einem durch dieses in der Urethra festlegbaren Ventilkörper (13) mit einem von außen durch Druckausübung manuell betätigbaren Ventil (17), **dadurch gekennzeichnet, dass** das Verankerungsteil (12) einen zylindrischen Hauptkörper (26) mit einem ersten Durchmesser (D) und an einem Ende desselben einen ebenfalls zylindrischen Endabschnitt (27) mit einem kleineren zweiten Durchmesser (d) und dass Hauptkörper (26) des Verankerungsteils (12) und Endabschnitt (27) desselben einen sie fest miteinander verbindenden, sich erweiternden Haltebereich (23; 30) für den Ventilkörper (13) aufweisen.

2. Endosphinkter nach Anspruch 1, **dadurch gekennzeichnet, dass** der Haltebereich (23; 31, 32, 33) schulterartig ausgebildet ist.

3. Endosphinkter nach Anspruch 1, **dadurch gekennzeichnet, dass** der Haltebereich (23; 31, 32, 33) einen sich vom Endabschnitt (27) trompetenartig erweiternden Erweiterungsabschnitt (31) und einen sich vom Hauptkörper (26) zum Erweiterungsabschnitt (31) hin bogenförmig eingezogenen Verjüngungsabschnitt (32) aufweist.

4. Endosphinkter nach Anspruch 3, **dadurch gekennzeichnet, dass** zwischen Erweiterungsabschnitt (31) und Verjüngungsabschnitt (32) ein sich teilkonisch erweiternder Zwischenabschnitt (33) ausgebildet ist.

5. Endosphinkter nach einem der Ansprüche 1 bis 4, **dadurch gekennzeichnet, dass** das Verankerungsteil (12) zum Festlegen des Ventilkörpers (13) in der Urethra in seiner Mantelwand Durchbrechungen (25) aufweist.

6. Endosphinkter nach Anspruch 5, **dadurch gekennzeichnet, dass** das Verankerungsteil (12) in seiner Einsatzkonfiguration rautenförmige Durchbrechungen (25) aufweist.

7. Endosphinkter nach einem der Ansprüche 1 bis 4, **dadurch gekennzeichnet, dass** das Verankerungsteil (12) schraubenfederartig ausgebildet ist.

8. Endosphinkter nach einem der Ansprüche 1 bis 7, **dadurch gekennzeichnet, dass** das Verankerungsteil (12) zum Festlegen des Ventilkörpers (13) in der Urethra aus Formgedächtnislegierung, wie Nitinol, besteht.

9. Endosphinkter nach einem der vorangehenden Ansprüche, **dadurch gekennzeichnet, dass** Verankerungsteil (12) und Ventilkörper (13) derart fest miteinander verbunden sind, dass sie nicht ohne Beschädigung voneinander trennbar sind.

10. Endosphinkter nach einem der Ansprüche 1 bis 8, **dadurch gekennzeichnet, dass** Verankerungsteil (12) und Ventilkörper (13) lösbar miteinander verbunden sind.

11. Endosphinkter nach einem der Ansprüche 1 bis 10, **dadurch gekennzeichnet, dass** das Verankerungsteil (12) eine Tieftemperaturkonfiguration mit geringen Durchmessern und eine Hochtemperaturkonfiguration oberhalb einer Temperatur von deutlich unterhalb 37 °C mit größeren Durchmessern als die der Tieftemperaturkonfiguration aufweist.

12. Endosphinkter nach einem der Ansprüche 1 bis 11, **dadurch gekennzeichnet, dass** das Verankerungsteil (12) bei einer Temperaturerhöhung über eine Übergangstemperatur hin, die deutlich unter 37 °C liegt, selbstaufweitend ist.

13. Endosphinkter nach einem der Ansprüche 1 bis 12, **dadurch gekennzeichnet, dass** der Ventilkörper (13) einen an das Verankerungsteil (12) angepassten Haltebereich (20) aufweist.

14. Endosphinkter nach Anspruch 13, **dadurch gekennzeichnet, dass** der Haltebereich (20) des Ventilkörpers (13) in radialer Richtung elastisch ausgebildet ist.

15. Endosphinkter nach einem der vorangehenden Ansprüche, **dadurch gekennzeichnet, dass** der Ventilkörper einen sich vom Haltebereich (20) erstreckenden schlauchförmigen zylindrischen Hauptkörper (19) mit geringerem Durchmesser als der Haltebereich (20) aufweist.

16. Endosphinkter nach Anspruch 15, **dadurch gekennzeichnet, dass** der zylindrische Hauptkörper (19) einen Durchmesser in der Größenordnung des zweiten Durchmessers (d) des Verankerungsteils (12) und der Haltebereich (20) einen Durchmesser in der Größenordnung des ersten Durchmessers des Verankerungsteils (12) aufweist.

17. Endosphinkter nach Anspruch 15 oder 16, **dadurch gekennzeichnet, dass** der zylindrische Hauptkörper (19) nahe dem Haltebereich (20) mit Dichtlippen (15) versehen ist.

18. Endosphinkter nach Anspruch 17, **dadurch gekennzeichnet, dass** die Dichtlippen (15) radial sich etwas über den Haltebereich (20) hinaus erstrecken.

19. Endosphinkter nach einem der Ansprüche 15 bis 18, **dadurch gekennzeichnet, dass** das Ventil (17) in einem dem Haltebereich (20) abgewandten Ende des zylindrischen Hauptkörper (19) ausgebildet ist.

20. Set zum freigebbaren Verschließen der Urethra mit einem künstlichen Endosphinkter nach einem der Ansprüche 1 bis 19 und mit einer Einrichtung zum Einführen des Ventilkörpers (13) in die Urethra mit einem Anschlag (64) als Gegenhalter für den Ventilkörper (13) und einem den Anschlag umgebenden, den Ventilkörper (13) aufnehmenden Kanülenrohr (62) zum Freigeben des Ventilkörpers aus diesem.

21. Set nach Anspruch 20, **gekennzeichnet durch** eine Einrichtung zum Vorabeinführen des Verankerungsteils (12) in die Urethra mit einem Gegenhalter (75) für das Verankerungsteil (12) und einem den Gegenhalter (75) umgebenden, das Verankerungsteil (12) aufnehmenden, relativ zum Gegenhalter (75) zurückziehbaren, äußeren Kanülenrohr (72) zum Freigeben des Verankerungsteils (12) in die Urethra.

22. Set nach Anspruch 21, **dadurch gekennzeichnet, dass** der Durchmesser des vorderen Endes des Kanülenrohres (72) kleiner ist als der Durchmesser (d) des Endabschnittes (27) des Verankerungsteils.

23. Set nach Anspruch 22, **dadurch gekennzeichnet, dass** innerhalb des äußeren Kanülenrohrs (62) ein den Ventilkörper umgebendes, aus dem äußeren Kanülenrohr (62) ausfahrbares Führungsrohr angeordnet ist, das zu seinem freien Ende in flexible Finger gespalten ist, deren freie Enden aufeinander zu gebogen sind.

## Claims

1. Artificial endosphincter for the urethra, having an anchoring part (12) and a valve body (13) fixable by the latter in the urethra and having a valve (17) manually operable from the outside by exerting pressure, **characterized in that** the anchoring part (12) has a cylindrical main body (26) with a first diameter (D) and on one end thereof an also cylindrical end portion (27) with a smaller, second diameter (d) and that the main body (26) of the anchoring part (12) and the end portion (27) thereof have a widening holding area (23; 30) for the valve body (13) firmly interconnecting the same.

2. Endosphincter according to claim 1, **characterized in that** the holding area (23; 31, 32, 33) has a shoulder-like construction.

3. Endosphincter according to claim 1, **characterized in that** the holding area (23; 31, 32, 33) has a widening portion (31) widening in trumpet-like manner from the end portion (27) and a narrowing portion (32) retracted arcuately towards the widening portion (31).

4. Endosphincter according to claim 3, **characterized in that** a partly conically widening intermediate portion (33) is formed between the widening portion (31) and the narrowing portion (32).

5. Endosphincter according to one of the claims 1 to 4, **characterized in that** the anchoring part (12) for fixing the valve body (13) in the urethra has openings (25) in its jacket wall.

6. Endosphincter according to claim 5, **characterized in that** in its use configuration the anchoring part (12) has diamond-shaped openings (25).

7. Endosphincter according to one of the claims 1 to 4, **characterized in that** the anchoring part (12) has a helical spring-like construction.

8. Endosphincter according to one of the claims 1 to 7, **characterized in that** the anchoring part (12) for fixing the valve body (13) in the urethra is made from memory alloy, such as nitinol.

9. Endosphincter according to one of the preceding claims, **characterized in that** the anchoring part (12) and valve body (13) are so firmly interconnected that they cannot be separated from one another without damage.

10. Endosphincter according to one of the claims 1 to 8, **characterized in that** the anchoring part (12) and valve body (13) are detachably interconnected.

11. Endosphincter according to one of the claims 1 to 10, **characterized in that** the anchoring part (12) has a low temperature configuration with small diameters and a high temperature configuration above a temperature of well below 37°C with larger diameters than that of the low temperature configuration.

12. Endosphincter according to one of the claims 1 to 11, **characterized in that** with a temperature increase above a transition temperature which is well below 37°C, the anchoring part (12) is self-expanding.

13. Endosphincter according to one of the claims 1 to 12, **characterized in that** the valve body (13) has a holding area (20) adapted to the anchoring part (12).

14. Endosphincter according to claim 13, **characterized in that** the holding area (20) of the valve body (13) is constructed elastically in the radial direction.

15. Endosphincter according to one of the preceding claims, **characterized in that** the valve body has a tubular, cylindrical main body (19) with a smaller diameter than the holding area (20) and extending away from the latter.

16. Endosphincter according to claim 15, **characterized in that** the cylindrical main body (19) has a diameter of the same order of magnitude as the second diameter (d) of the anchoring part (12) and the holding area (20) has a diameter of the same order of magnitude as the first diameter of the anchoring part (12).

17. Endosphincter according to claim 15 or 16, **characterized in that** the cylindrical main body (19) is provided with sealing lips (15) close to the holding area (20).

18. Endosphincter according to claim 17, **characterized in that** the sealing lips (15) extend somewhat radially over and beyond the holding area (20).

19. Endosphincter according to one of the claims 15 to 18, **characterized in that** the valve (17) is constructed in an end of the cylindrical main body (19) remote from the holding area (20).

20. Set for the releasable sealing of the urethra with an artificial endosphincter according to one of the claims 1 to 19 and with a device for introducing the valve body (13) into the urethra with a stop member (64) as a back stop for the valve body (13) and a cannula tube (62) surrounding the stop member and receiving the valve body (13) for the release of the latter therefrom.

21. Set according to claim 20, **characterized by** a device for the pre-insertion of the anchoring part (12) in the urethra with a back stop (75) for the anchoring part (12) and an external cannula tube (72), retractable relative to and surrounding the back stop (75) and receiving the anchoring part (12) for releasing the latter in the urethra.

22. Set according to claim 21, **characterized in that** the diameter of the front end of the cannula tube (72) is smaller than the diameter (d) of the end portion (27) of the anchoring part.

23. Set according to claim 22, **characterized in that** within the outer cannula tube (62) is received a guide tube extendable from the outer cannula tube (62) and surrounding the valve body and which towards its free end is split into flexible fingers, whose free ends are bent towards one another.

## Revendications

1. Endosphincter artificiel pour l'urètre, comprenant une pièce d'ancrage (12) et un corps de valve (13) pouvant être monté fixe par celle-ci dans l'urètre comprenant une valve (17) actionnable manuellement de l'extérieur par pression, **caractérisé en ce que** la pièce d'ancrage (12) présente un corps principal cylindrique (26) ayant un premier diamètre (D) et à une extrémité de celui-ci une extrémité (27) également cylindrique ayant un deuxième diamètre inférieur (d), et **en ce que** le corps principal (26) de la pièce d'ancrage (12) et l'extrémité (27) de celui-ci présentent une zone de maintien (23; 30) du corps de valve (13) qui les relie rigidement et va en s'élargissant.

2. Endosphincter selon la revendication 1, **caractérisé en ce que** la zone de maintien (23; 31,32,33) est conformée en épaulement.

3. Endosphincter selon la revendication 1, **caractérisé en ce que** la zone de maintien (23; 31,32,33) présente une partie d'élargissement (31) s'élargissant à la manière d'une trompette à partir de l'extrémité (27) et une partie de rétrécissement (32) se rétrécissant en forme d'arc à partir du corps principal (26) vers la partie d'élargissement (31).

4. Endosphincter selon la revendication 3, **caractérisé en ce qu'**entre la partie d'élargissement (31) et la partie de rétrécissement (32) est conformée une partie intermédiaire (33) s'élargissant de façon tronconique.

5. Endosphincter selon l'une quelconque des revendications 1 à 4, **caractérisé en ce que** la pièce d'ancrage (12) présente dans sa paroi d'enveloppe des ouvertures traversantes (25) pour la fixation du corps de valve (13) dans l'urètre.

6. Endosphincter selon la revendication 5, **caractérisé en ce que** la pièce d'ancrage (12) présente dans sa conformation d'insertion des ouvertures (25) en forme de losange.

7. Endosphincter selon l'une quelconque des revendications 1 à 4, **caractérisé en ce que** la pièce d'ancrage (12) est conformée en ressort cylindrique.

8. Endosphincter selon l'une quelconque des revendications 1 à 7, **caractérisé en ce que** la pièce d'ancrage (12) pour la fixation du corps de valve (13) dans l'urètre est réalisée dans un alliage à mémoire de forme tel le nitinol.

9. Endosphincter selon l'une quelconque des revendications précédentes, **caractérisé en ce que** la pièce d'ancrage (12) et le corps de valve (13) sont rigidement reliés entre eux de telle façon qu'on ne peut pas les séparer sans les endommager.

10. Endosphincter selon l'une quelconque des revendications 1 à 8, **caractérisé en ce que** la pièce d'ancrage (12) et le corps de valve (13) sont réalisés détachables l'un de l'autre.

11. Endosphincter selon l'une quelconque des revendications 1 à 10, **caractérisé en ce que** la pièce d'ancrage (12) présente une conformation de basse température à diamètres réduits et une conformation de haute température au-dessus d'une température se situant nettement en-dessous de 37°C à diamètres supérieurs à ceux de la conformation de basse température.

12. Endosphincter selon l'une quelconque des revendications 1 à 11, **caractérisé en ce que** la pièce d'ancrage (12) est auto-élargissante dans le cas d'une augmentation de la température au-delà d'une température de transition de résilience située nettement en-dessous de 37°C.

13. Endosphincter selon l'une quelconque des revendications 1 à 12, **caractérisé en ce que** le corps de valve (13) présente une zone de maintien (20) adaptée à la pièce d'ancrage (12).

14. Endosphincter selon la revendication 13, **caractérisé en ce que** la zone de maintien (20) du corps de valve (13) est conformée élastique en direction radiale.

15. Endosphincter selon l'une quelconque des revendications précédentes, **caractérisé en ce que** le corps de valve présente un corps principal (19) tubulaire cylindrique s'étendant à partir de la zone de maintien (20) de diamètre inférieur à celui de la zone de maintien (20).

16. Endosphincter selon la revendication 15, **caractérisé en ce que** le corps principal cylindrique (19) présente un diamètre du même ordre de grandeur que le deuxième diamètre (d) de la pièce d'ancrage (12) et **en ce que** la zone de maintien (20) présente un diamètre du même ordre de grandeur que le premier diamètre de la pièce d'ancrage (12).

17. Endosphincter selon la revendication 15 ou 16, **caractérisé en ce que** le corps principal (19) cylindrique est muni à proximité de la zone de maintien (20) de lèvres d'étanchéité (15).

18. Endosphincter selon la revendication 17, **caractérisé en ce que** les lèvres d'étanchéité (15) s'étendent radialement légèrement au-delà de la zone de maintien (20).

19. Endosphincter selon l'une quelconque des revendications 15 à 18, **caractérisé en ce que** la valve (17) est conformée dans une extrémité du corps principal (19) cylindrique opposée à la zone de maintien (20).

20. Ensemble pour la fermeture libérable de l'urètre comprenant un endosphincter artificiel selon l'une quelconque des revendications 1 à 19 et un dispositif d'introduction du corps de valve (13) dans l'urètre présentant une butée (64) en tant que contre-support pour le corps de valve (13) et une canule (62) entourant la butée et recevant le corps de valve (13) pour libérer le corps de valve de celle-ci.

21. Ensemble selon la revendication 20, **caractérisé par** un dispositif pour l'introduction préalable de la pièce d'ancrage (12) dans l'urètre présentant un contre-support (75) pour la pièce d'ancrage (12) et une canule (72) extérieure entourant le contre-support (75), recevant la pièce d'ancrage (12) et rétractable par rapport au contre-support (75) pour la libération de la pièce d'ancrage (12) dans l'urètre.

22. Ensemble selon la revendication 21, **caractérisé en ce que** le diamètre de l'extrémité avant de la canule (72) est inférieur au diamètre (d) de la section d'extrémité (27) de la pièce d'ancrage.

23. Ensemble selon la revendication 22, **caractérisé en ce qu'**à l'intérieur de la canule (62) extérieure est disposé un conduit de guidage entourant le corps de valve et déplaçable en-dehors de la canule extérieure (62), conduit fendu vers son extrémité libre en doigts flexibles dont les extrémités libres sont recourbées les unes vers les autres.
